# EUROPEAN PATENT SPECIFICATION

(11) **EP 2 346 501 B1**
(45) Date of publication and mention of the grant of the patent: **14.09.2016**
(21) Application number: 09756735.8
(22) Date of filing: 20.11.2009
(51) Int. Cl.: A23L 3/34, A61M 1/16, A61K 31/23, A61K 31/4164, A61P 31/04, A61P 31/10

(54) **USE OF CATIONIC SURFACTANTS FOR THE INACTIVATION OF TOXINS**
VERWENDUNG VON KATIONISCHEN TENSIDEN ZUR INAKTIVIERUNG VON TOXINEN
EMPLOI DE TENSIOACTIFS CATIONIQUES DANS L'INACTIVATION DE TOXINES

(30) Priority: 21.11.2008 US 116705 P
(43) Date of publication of application: 27.07.2011
(73) Proprietor: Vedeqsa Inc., New York, New York 10001 (US)
(72) Inventor: GAFFAR, Abdul, Princeton New Jersey 08540 (US); ROCABAYERA BONVILA, Xavier, E-08184 Palau-Solita / Barcelona (ES)
(74) Representative: Gille Hrabal
(86) International application number: PCT/EP2009/065524
(87) International publication number: WO 2010/057966

(56) References cited:
- WO-A1-03/013454
- WO-A1-03/034842
- WO-A1-03/043593
- US-A1- 2007 140 990
- US-A1- 2008 200 890
- MARTIN M DINGES ET AL: "Exotoxins of Staphylococcus aureus" CLINICAL MICROBIOLOGY REVIEWS, WASHINGTON, DC, US, vol. 13, no. 1, 1 January 2000 (2000-01-01), pages 16-34, XP007912189 ISSN: 0893-8512 [retrieved on 2010-03-12]
- RODRIGUEZ S B ET AL: "Inhibition of aflatoxin production by surfactants" APPLIED AND ENVIRONMENTAL MICROBIOLOGY, AMERICAN SOCIETY FOR MICROBIOLOGY, US, vol. 60, no. 1, 1 January 1994 (1994-01-01), pages 106-110, XP002518821 ISSN: 0099-2240
- DATABASE BIOSIS [Online] BIOSCIENCES INFORMATION SERVICE, PHILADELPHIA, PA, US; March 2006 (2006-03), KURONUMA KOJI ET AL: "Anionic surfactant phospholipids inhibit lipopolysaccaride-induced inflammation from alveolar macrophages and U937 cells" XP002573445 Database accession no. PREV200600342801
- IKEGAMI MACHIKO ET AL: "INTRATRACHEAL RECOMBINANT SURFACTANT PROTEIN D PREVENTS ENDOTOXIN SHOCK IN THE NEWBORN PRETERM LAMB" AMERICAN JOURNAL OF RESPIRATORY AND CRITICAL CARE MEDICINE, AMERICAN LUNG ASSOCIATION, NEW YORK, NY, US, vol. 173, no. 12, 23 March 2006 (2006-03-23), pages 1342-1347, XP009081677 ISSN: 1073-449X

## Description

### Field of the invention

This invention relates to the use of cationic surfactants with antimicrobial properties as agents for the inactivation of toxins.

### Background art

Endotoxins are complex macromolecules containing lipid, carbohydrate and protein. They are mainly found in the surface of Gram-negative organisms and are usually referred to as lipopolysaccharides (LPS). These macromolecules are toxic to the host and can be fatal. For instance, they can cause inflammation, severe hypotensive shock and elicit a variety of toxic reactions in the body. Sometimes LPS is released from bacteria which are dying either through the action of the body's natural defense system or due to the administration of antibiotics (Holzheimer, R.G., 2001: J. Chemother. 13:159-172).

Antimicrobial agents still represent the most important treatment of endotoxin-induced toxic reactions, but they are insufficient, since there is no effective endotoxin-neutralizing treatment which would inactivate and sequester LPS.

Lipopolyamines have recently shown promising results for neutralizing endotoxins. Lipopolyamines are polycationic amphiphilic molecules originally used as DNA transfection agents. These agents bind and neutralize LPS with affinity comparable to polymyxin B, a polycationic peptide antibiotic of microbial origin, which is one of the most potent LPS neutralizers, but has significant toxicity (David, S.A., 2001: J. Mol. Recognit. 14, 370-387; Jerala, R. & Porro, M., 2004. Curr.Top.Med.Chem. 4,1173-1184). US patent application 2007/0238655 describes the use of gelsolin therapy for the prevention of LPS-induced mortality.

US patent application 2007/0287750 describes certain lipophilic polycationic sulfonamides de-activating LPS. However, these compounds also induce lysis of red blood cells and have limited utility in vivo. Lipopeptides for neutralizing endotoxins are disclosed in Slovenian patent application SI 20877A. Antimicrobial peptides for neutralizing endotoxins are described in WO 2001/92290A2. This document refers to the peptide gomesin with 18 amino acid residues which possesses activity to lyze red blood cells. The use of antimicrobial agents such as taurolidine is described in WO 99/34805A2. These antimicrobial substances deactivate endotoxins by releasing formaldehyde which is toxic. Other peptide-based endotoxin-binding agents based on protegrin are disclosed in WO 97/18826A1. Similarly, naturally occurring protegrin to neutralize endotoxin from Gram-negative bacteria are described in Journal of Infection and Chemotherapy 1997, Vol (1), pp 27-32 by Sawada, H.

The prior art describes agents for binding and inactivating endotoxins which have several deficiencies for use in food, cosmetics, beverages and pharmaceutical preparations. Most of them induce lysis of red blood cells, release toxic by-products or are expensive to manufacture. Thus, there is a large interest to obtain novel agents to inactivate endotoxins that overcome the disadvantages of these substances.

Further toxic products excreted by microorganisms are exotoxins and aflatoxins. With these types of toxins there exists the same unsatisfactory situation of not having the tools for inactivating these products.

An exotoxin is a soluble protein excreted by a microorganism, including bacteria, fungi, algae and protozoa. An exotoxin can cause damage to the host by destroying cells or disrupting the normal cellular metabolism. Gram-negative as well as Gram-positive bacteria produce exotoxins. They are highly potent and can cause major damage to the host. Exotoxins may be secreted, or, similar to endotoxins, may be released during lysis of the cell.

Most exotoxins can be destroyed by heating. They may exert their effect locally or produce systemic effects. Well-known exotoxins include the botulinum toxin produced by *Clostridium botulinum* and the *Corynebacterium diphtheriae* exotoxin which is produced during life threatening symptoms of diphtheria.

Exotoxins are susceptible to antibodies produced by the immune system, but many exotoxins are so toxic that they may be fatal to the host before the immune system has a chance to mount defenses against it.

Aflatoxins are naturally occurring mycotoxins that are produced by many species of Aspergillus, a fungus, most notably *Aspergillus flavus* and *Aspergillus parasiticus.* Aflatoxins are toxic and among the most carcinogenic substances known. After entering the body, aflatoxins are metabolized by the liver to a reactive intermediate, aflatoxin M₁, an epoxide.

Cationic surfactants are known as preservatives used in food, cosmetic and pharmaceutical industry. Cationic surfactants have turned out to be highly effective against microbial proliferation and at the same time safe for intake in humans and mammals in general. For all of this, cationic surfactants are an attractive tool in the industry.

It has been demonstrated that cationic surfactants according to formula (1) derived from the condensation of fatty acids and esterified dibasic amino acids are highly effective protective substances against microorganisms. 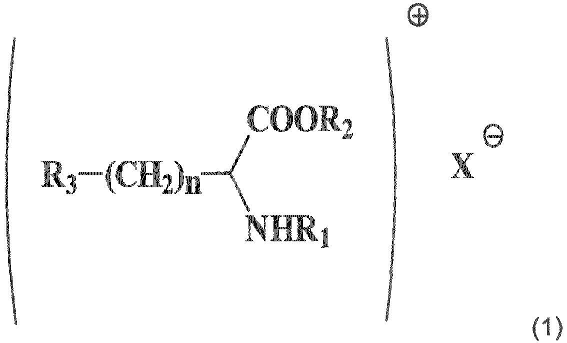 where:
X⁻ is a counter ion derived from an organic or inorganic acid, preferably Br⁻, Cl⁻ or HSO₄⁻, or an anion on the basis of a phenolic compound;
R₁: is a straight alkyl chain from a saturated fatty acid or hydroxyl acid having from 8 to 14 atoms linked to the α-amino acid group via an amidic bond;
R₂: is a straight or branched alkyl chain from 1 to 18 carbon atoms or an aromatic group;
R₃: is

   —NH₃

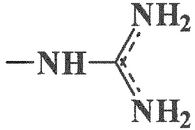

   or 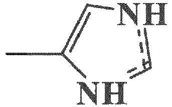
where n is from 0 to 4.

The organic acids which may be the source of the counter ion X⁻ can be citric acid, lactic acid, acetic acid, fumaric acid, maleic acid, gluconic acid, propionic acid, sorbic acid, benzoic acid, carbonic acid, glutamic acid or other amino acids, lauric acid and fatty acids such as oleic acid and linoleic acid, whereas the inorganic acids can be phosphoric acid, nitric acid and thiocyanic acid.

The phenolic compound which may be the basis of the anion X⁻ is for instance butylated hydroxyanisole (BHA) and the related butylated hydroxytoluene, tertiary butyl hydroquinone and parabens such as methylparaben, ethylparaben, propylparaben and butylparaben.

The most preferred compound of the above class of compounds is the ethyl ester of the lauramide of the arginine monohydrochloride, hereafter referred to as LAE (CAS No. 60372-77-2). This compound is now well-known for its use as an antimicrobial agent. In practical use LAE turned out to be well tolerated and to display a very low toxicity to human beings. LAE has the chemical structure of formula (2) displayed hereafter.

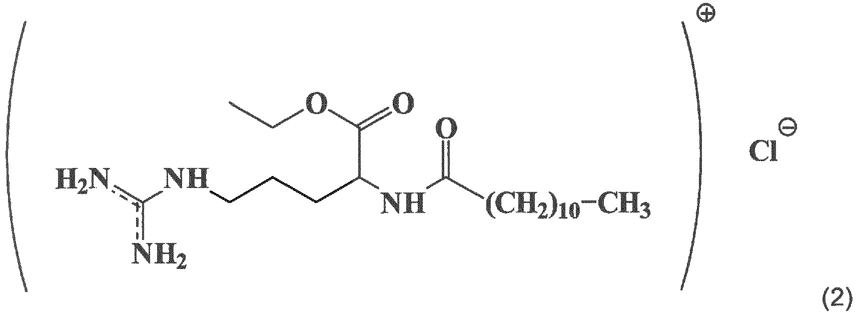

The compound LAE is remarkable for its activity against different microorganisms, like bacteria, moulds and yeasts which can be present in food products (WO 03/034842) and also in cosmetic formulations and preparations (WO 03/013453, WO 03/013454 and WO 03/043593). The compound has been furthermore described for its effect on parasites in fish, such as on the larvae of Anisakis or other species (European application 07 382 004.5). Its preservative action is particularly pronounced in a combination with a polyene fungicide such as natamycin (PCT/EP2007/060598). It has furthermore been shown to be effective for killing endospores and for having an effect in virus infections (European application 08 382 025.8). The specific use for the protection of teeth against dental erosion has been described (European application 08 382 007.6).

The general preparation of the cationic surfactants is described in Spanish patent ES 512643 and international patent applications WO 96/21642, WO 01/94292 and WO 03/064669.

LAE, also known as lauric arginate, is manufactured by Laboratories Miret, S.A. (LAMIRSA, Spain). Lauric arginate is listed by the FDA (Food and Drug Administration) as being a GRAS substance (Generally Recognized As Safe) under GRN 000164. The USDA (United States Department of Agriculture) has approved its use in meat and poultry products (FSIS Directive 7120.1) and also as a processing aid for fresh meat and poultry products.

The metabolism of the above cationic surfactant of formula (2) in rats has been studied; these studies have shown a fast absorption and metabolisation into naturally-occuring amino acids and the fatty acid lauric acid, which are eventually excreted as carbon dioxide and urea. Toxicological studies have demonstrated that LAE is completely harmless to animals and humans.

Therefore, LAE and related compounds are particularly suitable to be used in the preservation of all perishable food products. LAE and related compounds are equally suitable for use in cosmetic or medical products and in medical devices where growth of microorganisms is common.

As has been remarked above, the cationic surfactants are remarkable for their inhibitory action over the proliferation of different microorganisms, such as bacteria, fungi and yeasts. The minimum inhibitory concentrations of LAE are shown in the following table 1.

**Table 1**

| **Kind** | **Microorganism** | **M.I.C. (ppm)** |
|---|---|---|
| **Gram + Bacteria** | *Arthrobacter oxydans* ATCC 8010 | 64 |
| | *Bacillus cereus var mycoide* ATCC 11778 | 32 |
| | *Bacillus subtilis* ATCC 6633 | 16 |
| | *Clostridium perfringens* ATCC 77454 | 16 |
| | *Listeria monocytogenes* ATCC 7644 | 10 |
| | *Staphylococcus aureus* ATCC 6538 | 32 |
| | *Micrococcus luteus* ATCC 9631 | 128 |
| | *Lactobacillus delbrueckii ssp lactis* CECT 372 | 16 |
| | *Leuconostoc mesenteroides CETC 912* | 32 |
| **Gram - Bacteria** | *Alcaligenes faecalis* ATCC 8750 | 64 |
| | *Bordetella bronchiseptica* ATCC 4617 | 128 |
| | *Citrobacter freundii* ATCC 22636 | 64 |
| | *Enterobacter aerogenes* CECT 689 | 32 |
| | *Escherichia coli* ATCC 8739 | 32 |
| | *Escherichia coli* 0157H7 | 20 |
| | *Klebsiella pneumoniae var pneumoniae* CECT | 32 |
| | 178 | 32 |
| | *Proteus mirabilis* CECT 170 | 64 |
| | *Pseudomonas aeruginosa* ATCC 9027 | 32 |
| | *Salmonella typhimurium* ATCC16028 | 32 |
| | *Serratia marcenses* CECT 274 | 2 |
| | *Mycobacterium phlei* ATCC 41423 | |
| Fungi | *Aspergillus niger* ATCC14604 | 32 |
| | *Aureobasidium pullulans* ATCC 9348 | 16 |
| | *Gliocadium virens* ATCC 4645 | 32 |
| | *Chaetonium globosum* ATCC 6205 | 16 |
| | *Penicillium chrysogenum* CECT 2802 | 128 |
| | *Penicillium funiculosum* CECT 2914 | 16 |
| Yeast | *Candida albicans* ATCC 10231 | 16 |
| | *Rhodotorula rubra* CECT 1158 | 16 |
| | *Saccharomyces cerevisiae* ATCC 9763 | 32 |

It is preferred to dissolve the compound directly before use in one of the following preferred solvents of food grade: water, ethanol, propylene glycol, isopropyl alcohol, other glycols, mixtures of glycols and mixtures of glycols and water, diacetin, triacetin, glycerol, sorbitol, mannitol and xylitol. If the treatment shall be performed at a specific pH value, the use of a corresponding buffer solution may be recommendable. On the other hand, the compound can be easily used in its solid form or formulated with solid carriers such as salt, sugar, maltodextrine, hydrocolloids and sorbitol.

For the cationic surfactants of the above formula (1) the antibacterial activity and the biological activity against other microorganisms such as fungi and yeasts is well documented.

### Summary of the invention

Subject-matter which is not encompassed by the scope of the claims does not form part of the presently claimed invention.

It is the object of the present invention to provide a novel method for inactivating the effects of toxins in food products and beverages and on surfaces of medical equipment.

More in particular, it is the object of the present invention to provide a novel method for inhibiting endotoxins (LPS) released from Gram-negative bacteria in order to reduce or mitigate the damages caused by LPS in animals and human beings who ingest food products or beverages which are contaminated with endotoxins, or who have been in contact with surfaces of medical equipment which are contaminated.

It is the further object of the present invention to provide a novel method for inhibiting exotoxins and aflatoxins in food products and beverages and on surfaces of medical equipment.

The inventors of the present invention have surprisingly observed that the objects of the present invention can be solved by using the cationic surfactants derived from the condensation of fatty acids and esterified dibasic amino acids of the above formula (1). It has been observed, that the direct addition of these cationic surfactants derived from the condensation of fatty acids and esterified dibasic amino acids of the above formula (1) to food products and beverages causes inactivation of the endotoxins. It has been observed that the cationic surfactants derived from the condensation of fatty acids and esterified dibasic amino acids of the above formula (1) can lead to a reduction of inflammation and an improvement of hypotensive shock after contact with endotoxins and accordingly the cationic surfactants are suitable for use in a method of medical treatment for the in-vivo inactivation of endotoxins which are released from Gram-negative bacteria. Similar beneficial effects have been observed in the interaction with exotoxins and aflatoxins.

### Description of the preferred embodiments

The most preferred compound of the above class of compounds is the ethyl ester of the lauramide of the arginine monohydrochloride, hereafter referred to as LAE (CAS No. 60372-77-2). This compound is particularly effective for the inactivation of toxins. LAE has the chemical structure of formula (2) displayed hereafter.

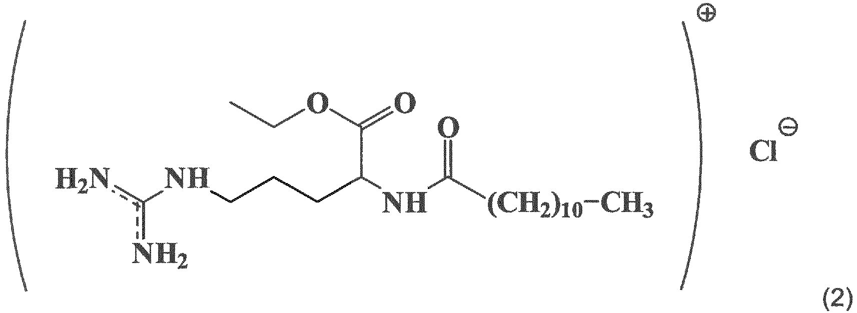

The cationic surfactants of the general formula (1) have been described as antimicrobial agents.

The cationic surfactants are regularly used as preservative agents in such products as food products and medical and cosmetic preparations or for medical devices.

It is particularly preferred to use the cationic surfactants of general formula (1) for the preservation of meat products, like for instance meat, poultry products, fish, crustaceans, vegetables, greens, emulsions, sauces, confectionery, bakery, dairy products, egg-based products, jams, jellies, beverages, juices, wines and beers.

Surprisingly, the inventors have now found LAE (N-alpha-lauroyl-L-arginine ethyl ester monohydrochloride) and its homologues to bind and neutralize LPS (lipopolysaccharides). Because of the low toxicity of LAE for use in food, beverages, cosmetics and pharmaceutical applications, this surprising finding has significant practical value for neutralizing and detoxifying the toxins produced by pathogenic bacteria.

LAE and its homologues described herein can be added directly to food, meat, beverages, poultry products, fish and crustaceans (shrimps, shell fish) to inactivate toxins at the concentration of concentration in the food of 0.1 mg/kg to 100,000 mg/kg, preferably at 1 mg/kg to 10,000 mg/kg, and most preferably at 5 mg/kg to 1000 mg/kg.

LAE and its homologues described herein can be administered to animals or human patients by any conventional method available for use, in conjunction with excipients, pharmaceuticals, either as individual therapeutic agents or in a combination of therapeutic agents.

The dose administered to an animal, particularly a human, in the context of the present invention should be sufficient to affect a therapeutic response in the animal or the human patient over a reasonable time frame. One skilled in the art will

recognize that dosage will depend upon a variety of factors including a condition of the animal, the body weight of the animal, as well as the condition being tested.

A suitable dose is that which will result in a concentration of the active agent in a patient which is known to affect the desired response.

The size of the dose will be determined by the route, timing and frequency of administration as well as the existence, nature, and extent of any adverse side effects that might accompany the administration of the compound and the desired physiological effect.

Useful pharmaceutical dosage forms for administration of the compounds according to the present invention can be illustrated as follows:

### Hard Shell Capsules

A large number of unit capsules are prepared by filling standard two-piece hard gelatin capsules each with 1000 mg of powdered active ingredient, 150 mg of lactose, 50 mg of cellulose and 6 mg of magnesium stearate.

### Soft Gelatin Capsules

A mixture of active ingredient in a digestible oil such as soybean oil, cottonseed oil or olive oil is prepared and injected by means of a positive displacement pump into molten gelatin to form soft gelatin capsules containing 100 to 1000 mg of the active ingredient. The capsules are washed and dried. The cationic surfactants can be dissolved in a mixture of polyethylene glycol, glycerine and sorbitol to prepare a water miscible medicine mix.

The cationic surfactants of formula (1), more in particular LAE as the preferred type of cationic surfactant, can be administered orally in solid dosage forms, such as capsules, tablets, and powders or in liquid dosage forms, such as elixirs, syrups and suspensions. It can also be administered parenterally, in sterile liquid dosage forms. The cationic surfactants of formula (1) can also be administered intranasally (nose drops) or by inhalation of a drug powder mist. Other dosage forms are potentially possible such as transdermal administration, via a patch mechanism or ointment. The active ingredient can be administered employing a sustained or delayed release delivery system or an immediate release delivery system.

The dosage administered to patients will, of course, vary depending upon known factors such as the pharmacodynamic characteristics of the particular agent and its mode and route of administration; the age, health and weight of the recipient; the nature and extent of the symptoms; the kind of concurrent treatment; the frequency of treatment; and the effect desired. A daily dosage of active ingredient of cationic surfactant of formula (1) by oral administration can be expected to be about 0.01 mg/kg of bodyweight to 900 mg/ kilogram of body weight, preferably 0.05 mg/kg of bodyweight to 90 mg/kg of bodyweight and more preferably 0.1 mg/kg of bodyweight to 9 mg/kg of bodyweight.

Dosage forms for oral administration (compositions suitable for administration) contain 1 to 500 mg of active ingredient of the cationic surfactant per unit. In these pharmaceutical compositions, the active ingredient will ordinarily be present in an amount of 0.05% - 95% weight based on the total weight of the composition.

Formulations suitable for oral administration can consist of (a) liquid solutions, such as an effective amount of the compound dissolved in diluents, such as water, saline or orange juice; (b) capsules, sachets, tablets, lozenges, and troches, each containing a predetermined amount of the active ingredient, as solids or granules; (c) powders; (d) suspensions in an appropriate liquid; and (e) suitable emulsions. Liquid formulations may include diluents, such as water and alcohols, for example, ethanol, benzyl alcohol, propylene glycol, glycerin, and the polyethylene alcohols, either with or without the addition of a pharmaceutically acceptable surfactant, suspending agent, or emulsifying agent. Capsule forms can be of the ordinary hard- or soft-shelled gelatin type containing for example, surfactants, lubricants and inert fillers, such as lactose, sucrose, calcium phosphate and corn starch. Tablet forms can include one or more of the following: lactose, sucrose, mannitol, corn starch, potato starch, alginic acid, microcrystalline cellulose, acacia, gelatin, guar gum, colloidal silicon dioxide, croscarmellose sodium, talc, magnesium stearate, calcium stearate, zinc stearate, stearic acid, and other excipients, colorants, diluents, buffering agents, disintegrating agents, moistening agents, preservatives, flavoring agents, and pharmacologically compatible carriers. Lozenge forms can comprise the active ingredient in a flavor, usually sucrose and acacia or tragacanth, as well as pastilles comprising the active ingredient in an inert base, such as gelatin and glycerin, or sucrose and acacia, emulsions, and gels containing, in addition to the active ingredient, such carriers as are known in the art.

The compounds of the present disclosure, alone or in combination with other suitable components, can be made into aerosol formulations to be administered via inhalation. These aerosol formulations can be placed into pressurized acceptable propellants such as dichloro-difluoromethane, propane, and nitrogen. They also may be formulated as pharmaceuticals for non-pressured preparations, such as in a nebulizer or an atomizer.

The dosage upon parenteral administration will be in the range of 0.01 to 900 mg/kg bodyweight, preferably 0.05 to 90 mg/kg bodyweight and most preferably 0.1 to 9 mg/kg bodyweight.

Formulations suitable for parenteral administration include aqueous and non-aqueous, isotonic sterile injections solutions, which can contain anti-oxidants, buffers, bacteriostats, and solutes that render the formulation isotonic with the blood of the intended recipient, and aqueous and non-aqueous sterile suspensions that can include suspending agents, solubilizers, thickening agents, stabilizers, and preservatives. The compound can be administered in physiologically acceptable diluents in a pharmaceutical carrier such as sterile liquid or mixture of liquids including water, saline, aqueous dextrose and related sugar solutions, an alcohol, such as ethanol, isopropanolol, or hexadecyl alcohol, glycols, such as propylene glycol or polyethylene glycol such as poly(ethyleneglycol)400, glycerol ketals such as 2,2-dimethyl-1-3-dioxolane-4-methanol, ethers, an oil, a fatty acid, a fatty acid ester or glyceride, or an acetylated fatty acid glyceride with or without the addition of a pharmaceutically acceptable surfactant, such as a soap or a detergent, suspending agent, such as pectin, carbomers, methylcellulose, hydroxypropyl-methylcellulose, or carboxymethylcellulose, or emulsifying agents and other pharmaceutical adjuvants. Oils which can be used in parenteral formulations include petroleum, animal, vegetable, or synthetic oils. Specific examples of oils include peanut, soybean, sesame, cottonseed, corn, olive, petrolatum, and mineral. Suitable fatty acids for use in parenteral formulations include oleic acid, stearic acid, and isostearic acid. Ethyl oleate and isopropyl myristate are examples of suitable fatty acid esters. Suitable soaps, for use in parenteral formulations include fatty alkali metal, ammonium, and triethanolamine salts and suitable detergents include (a) cationic detergents such as, for example dimethyldialkylammonium halides, and alkylpyridinium halides; (b) nonionic detergents such as for example, fatty amine oxides, fatty acid alkanolamides, and polyoxyethylene polypropylene copolymers; (c) amphoteric detergents such as, for example, alkylbetaaminopropionates, and 2-alkylimidazoline quaternary ammonium salts; and (d) mixtures thereof.

The parenteral formulations typically contain from 0.1 % to 25% by weight of the cationic surfactant in solution. Suitable preservatives and buffers can be used in such formulations. In order to minimize or eliminate irritation at the site of injection, such compositions may contain one or more nonionic surfactants having a hydrophile-lipophile balance (HLB) of from 12 to 17. The quantity of such surfactants in the formulations ranges from 5% to 15% by weight. Suitable nonionic surfactants having a hydrophile-lipophile balance (HLB) of from 12 to 17 include polyethylene sorbitan fatty acid esters, such as sorbitan monooleate and the high molecular weight adducts of ethylene oxide with a hydrophobic base, formed by the condensation of propylene oxide with propylene glycol.

Pharmaceutically acceptable excipients are also well-known to those who are skilled in the art. The choice of excipient will be determined in part by the particular compound, as well as by the particular method used to administer the composition. Accordingly, there is a wide variety of suitable formulations of the pharmaceutical composition of the present invention. The following methods and excipients are merely exemplary and are in no way limiting. The pharmaceutically acceptable excipients preferably do not interfere with the action of the active ingredients and do not cause adverse side effects. Suitable carriers and excipients include solvents such as water, alcohol, and propylene glycol, solid absorbants and diluents, surface active agents, suspending agents, tableting binders, lubricants, flavors, and coloring agents. The formulations can be presented in unit-dose or multi-dose sealed containers, such as ampoules and vials, and can be stored in a freeze-dried (lyophilized) condition requiring only the addition of the sterile liquid excipients, for example, water for injections, immediately prior to use. Extemporaneous injection solutions and suspensions can be prepared from sterile powders, granules and tablets. The requirements for effective pharmaceutical carriers for injectable compositions are well known to those of ordinary skill in the art, see Pharmaceutics and Pharmacy Practice, J.B. Lippincott Co., Philadelphia, PA., Banker and Chalmers, Eds., 238-250 (1982) and ASHP Handbook on Injectable Drugs, Toissel, 4th ed., 622-630 (1986). Formulations suitable for topical administration include lozenges comprising the active ingredient in a flavor, usually artificial sweetener such as sucralose and saccharin and acacia and tragacanth; pastilles comprising the active ingredient in an inert base, such as gelatin and glycerin, or sucrose and acacia; and mouth washes comprising the active ingredient in a suitable liquid carrier; as well as creams, emulsions, and gels containing, in addition to the active ingredient, such carriers as are known in the art.

LAE can be applied as dry powder or liquid form or cream formulation with wound dressing at amounts of 0.01 mg/kg of bodyweight to 2000 mg/kg of bodyweight to prevent sepsis and promote healing, preferred amounts being 0.05 mg/kg bodyweight to 500 mg/kg bodyweight and most preferred 0.1 mg/kg bodyweight to 50 mg/kg of bodyweight.

Additionally, formulations suitable for rectal administration may be presented as suppositories by mixing with a variety of bases such as emulsifying bases or water-soluble bases. Formulations suitable for vaginal administration may be presented as pessaries, tampons, creams, gels, pasts, foam or spray formulas containing, in addition to the active ingredient, such carriers as are known in the art to be appropriate.

The cationic surfactants of the formula (1), more in particular LAE of formula (2), can be used for the treatment of any surface which may get into contact with any of the toxins described in the present application, such as the endotoxins, the exotoxins and aflatoxins. This specific use may be particularly useful for such surfaces which are part of medical equipment, such as for instance catheters or other infusion equipment. The treatment of the surfaces of the catheters and other infusion equipment with the cationic surfactants of the formula (1) leads to the highly favourable effect of inactivating any endotoxins which may be present in the medium which flows through the catheter or other infusion equipment. The suitable dose level is between 0.2% and 1 % of cationic surfactant attached to the surface of catheters or other medical devices. In the same manner it may inactivate any exotoxin or aflatoxin which may be present. This is a particularly preferred embodiment of the present invention.

The utility of this invention will be obvious from the following illustrative examples. Any range of the values given herein may be extended or changed without losing the effects which are sought, as will be apparent to the skilled person with an understanding of the teaching herein.

### EXAMPLE 1

### Effect of different LAE -concentrations on endotoxins produced by E. coli 0113:H10.

### Objective:

The first objective of this experiment was to evaluate and compare two LAL assays (turbidimetric and pyrochrome assay). The second objective of this preliminary experiment was to determine the effect of LAE on endotoxins produced by *E. coli* O113:H10 that is used as a standard. The activity of the endotoxin treated with LAE was assessed via both methods.

### Materials and Methods:

Turbidimetric LAL test was conducted according to the instructions provided by the manufacturer (Pyrotell-T, The associates of Cape Cod, Inc.). The standard curve was built based on 300, 30, 3, 0.3, 0.03 and 0.003 EU/ml. The same endotoxin in concentrations of 12.5 µg/ml was used in the experiment.

LAE was applied in concentrations of 0, 5, 10, 20, 30, 50 µg/ml to assess this compound's ability to deactivate endotoxins. LAL reagent water (LRW) was used to make all the endotoxin dilutions including the standards. MIRENAT-CF is a 10.5% solution of LAE in Propylene glycol, so the dilutions of MIRENAT-CF were initially made in propylene glycol, so that concentration of LAE was kept as the only variable. For the presumptive deactivation procedure, the endotoxin was incubated together with LAE for two hours at 37° C. The turbidimetric assay was conducted two times using the onset time for OD405=0.05. The pyrochrome assay was conducted once using the same template on plates.

### Results:

**Effect of different LAE concentrations on endotoxins produced by *E. coli* O113:H10.**

**Table 2.**

| **CONCENTRATION LAE (µg/ml)** | **CONCENTRATION Endotoxin (EU/ml x10E3)** |
|---|---|
| **0** | **120** |
| **10** | **80** |
| **50** | **61** |

| | |
|---|---|
| LAE at 50 µg/ml reduced the endotoxin activity by 50% | |

The results of the two assays were similar, however the pyrochrome assay was not as sensitive at lower concentrations of endotoxin.

### The effect of propylene glycol on the recovery of endotoxins through the LAL assay.

### Objective:

Propylene glycol (PG) is used as a solvent for LAE in the MIRENAT products. This compound will be used as a negative control in the experiments; but it may interfere with the endotoxin recovery process through the LAL assay. Technical support at the Associates of Cape Cod stated that PG has been listed as an interfering substance, however, in the concentrations that this compound is used within the plate (1 µg/ml) it should not interfere with the assay. In this experiment we test the effect of PG on the recovery of endotoxins.

### Materials and methods:

500 µl of the *S*. *typhimurium* LPS (range 50-0.4 µg/ml) was mixed with either 500 µl of 1mg/ml solution of the PG or with 500 µl of sterile distilled water used to make the dilutions. Each mixture was incubated for two hours at 37 °C and then serially diluted within the glass reaction tubes using the LAL Reagent water. Turbidimetric LAL assay was then used to evaluate the recovery of endotoxins after the PG treatment. The experiment was repeated two times in duplicates.

### Results

Propylene glycol, in the concentrations used for the assay, does not interfere with the recovery of LPS.

### EXAMPLE 2

### The investigation of endotoxin deactivation response as a function of LAE Concentration with Salmonella typhimurium

### Objective:

The objective of this experiment was to build the dose response curve for the concentrations of LAE in the range of 5-50 µg/ml (50-500 ppm of MIRENAT-CF preparation).

### Materials and methods:

The 1 mg/ml stock solution of *S*. *typhimurium* LPS was prepared using the commercially available LPS obtained from the List Biological Laboratories, Inc. (cat # 225). The 20 µg/ml solution of LPS was prepared by serially diluting the stock. All the LPS solutions were kept at refrigeration temperatures according to the instructions provided by the manufacturer.

The solutions of LAE prepared were used in the experiment. For the procedure, 500 µl of 20 µg/ml of endotoxins were mixed with 500 µl of LAE solution (10, 20, 40, 60 and 100 µg/ml) in glass reaction tubes, giving the final concentration of LPS, 10 µg/ml and the concentration range of LAE, 5-50 µg/ml. As a negative control, LPS was mixed with 1 mg/ml aqueous solution of propylene glycol. The mixture was incubated for 2 hours at 37 °C. Each sample was serially diluted using the LAL reagent water to be assayed for the endotoxin activity.

### Results and discussion:

All three dilutions of every sample used in the plate (1000X, 10,000X, 100,000X) were within the range of the standard curve. The results were adjusted using the appropriate dilution factor. The standard curve itself followed a good trend.

LAE in concentrations of 5-50 µg/ml significantly decreased the activity of *S*. *typhimurium* endotoxins.

The results indicated that LAE in the form of MIRENAT-CF decreased the activity of endotoxins from S. *typhimurium* as detected by the LAL assay.

**Table 3.**

| **CONCENTRATION LAE (µg/ml)** | **CONCENTRATION Endotoxin (EU/ml X10E3)** |
|---|---|
| **0** | **17000** |
| **5** | **4000** |
| **10** | **3995** |
| **20** | **3000** |
| **30** | **4000** |
| **50** | **2500** |

This deactivation effect, however, does not follow a dose response for the investigated concentration range of the LAE (5-50 µg/ml).

The results obtained in the examples 1 and 2 clearly demonstrate the surprising ability of LAE at very low concentrations to inactivate endotoxins from Salmonella and E.coli.

### EXAMPLE 3

### Effect of LAE on LPS Activity on Human Cell Culture Systems.

To asses whether LAE is able to bind endotoxins and neutralizes their cytoxotic effect, the following experiment was performed. The toxicity of endotoxins was measured using human cells.

Human epithelial cell lines (KB) were obtained from American Cell type collection and grown in BGJ medium (Gibco, Buffalo, NY) at 37° C with 5% CO₂. The medium was supplemented with 5% heated calf serum. Cells were placed in 24 well dishes with 0.5 ml medium per well. The release of peptidases from the human cell lines in response to LPS was measured by a coumarin based fluorigenic substrate utilized to study eukaryotic enzymes that degrade extracellular matrix proteins (collagen and fibronectin) and inactivated by inhibitors of matrix of metalloprotease (Yang et al. Abstract 105 at the 105^{th} ASM general meeting).

### Results and Discussion

When the human cells are exposed to LPS, cells release peptidases indicating that there is cell damage. The release of peptidase was measured by sensitive fluorescence test.

The results depicted below in the table 4 indicate the dose-dependent effect of LPS on peptidase induction.

The release of peptidase for an untreated sample was of 400 units of fluorescence.

Addition of LAE at a concentration of 2 µg/ml to LPS resulted in significant reduction of peptidase response at both doses of LPS tested.

**Table 4.**

| **Conc. LAE (µg/ml)** | **Conc. LPS (µg/ml)** | **Peptidase released (Fluorescence units)** |
|---|---|---|
| **0 (blank sample)** | **0** | **400** |
| **2** | **0** | **296** |
| **0** | **10** | **900** |
| **2** | **10** | **580** |
| **0** | **20** | **1200** |
| **2** | **20** | **655** |

Exposing cells to 10 µg/ml or 20 µg/ml of LPS caused the release of peptidase. When the LPS was pre-mixed with 2 µg/ml of LAE, the toxicity of LPS (measured by fluorescence release) was surprisingly reduced in 35.6% for the pre-mix with 10 µg/ml LPS and 45.4% for the pre-mix with 20 µg/ml LPS.

At a very low concentration, 2 µg LAE /ml were effective in reducing the toxicity of endotoxin to human cells. This low concentration was chosen to ensure that LAE per se did not provoke the release of the enzymes since at 2 µg LAE /ml resulted in the release of peptidase less than 300 units, similar to the blank. Therefore the effect is due to binding of LAE to LPS to reduce toxicity.

## Claims

1. Use of a cationic surfactant derived from the condensation of fatty acids and esterified dibasic amino acids, according to the following formula (1): 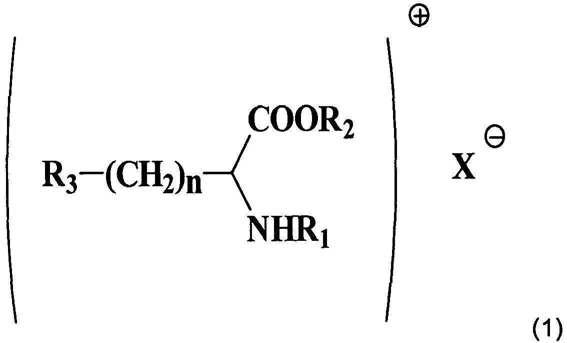 wherein
X- is a counter ion derived from an organic or inorganic acid, preferably Br⁻, Cl⁻ or HSO₄⁻, or an anion on the basis of a phenolic compound;
R₁: is a straight alkyl chain from a saturated fatty acid or hydroxyl acid having from 8 to 14 carbon atoms linked to the α-amino acid group via an amidic bond;
R₂: is a straight or branched alkyl chain from 1 to 18 carbon atoms or an aromatic group;
R₃: is
—NH₃ 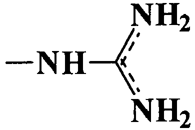
or 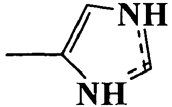
where n is from 0 to 4,
for the inactivation of endotoxins (lipopolysaccharides, LPS) released from Gram-negative bacteria, exotoxins or aflatoxins in food products and beverages.

2. The use according to claim 1 wherein the cationic surfactant is added to a food product at a concentration from 0.1 to 100,000 mg/kg of the food product, preferably from 1 to 10,000 mg/kg of food product and most preferably 5 to 1000 mg/kg of food product.

3. Use of a cationic surfactant derived from the condensation of fatty acids and esterified dibasic amino acids, according to the following formula (1): 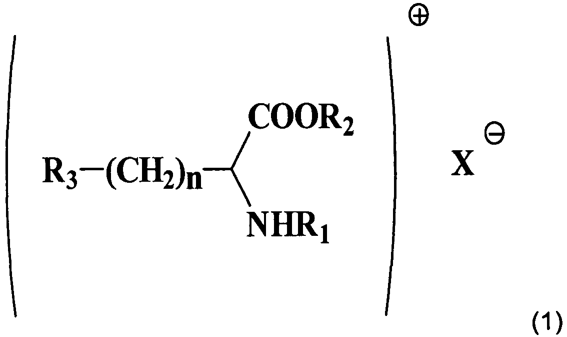 wherein
X⁻ is a counter ion derived from an organic or inorganic acid, preferably Br⁻, Cl⁻ or HSO₄⁻, or an anion on the basis of a phenolic compound;
R₁: is a straight alkyl chain from a saturated fatty acid or hydroxyl acid having from 8 to 14 carbon atoms linked to the α-amino acid group via an amidic bond;
R₂: is a straight or branched alkyl chain from 1 to 18 carbon atoms or an aromatic group;
R₃: is
—NH₃ 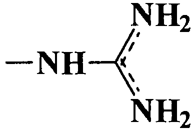
or 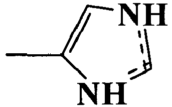
where n is from 0 to 4,
for the inactivation of endotoxins (lipopolysaccharides, LPS) released from Gram-negative bacteria, exotoxins or aflatoxins on surfaces of medical equipment.

4. The use according to claim 3 in a method for the treatment of medical devices by coating them with cationic surfactants attached to their surfaces at dose levels between 0.2% and 1 %.

5. The use according to any of claims 1 to 4, wherein the compound is the ethyl ester of the lauramide of arginine hydrochloride (LAE).

6. A cationic surfactant derived from the condensation of fatty acids and esterified dibasic amino acids, according to the following formula (1): 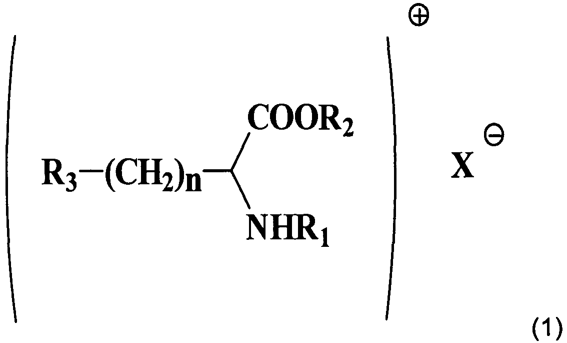 wherein
X⁻ is a counter ion derived from an organic or inorganic acid, preferably Br⁻, Cl⁻ or HSO₄⁻, or an anion on the basis of a phenolic compound;
R₁: is a straight alkyl chain from a saturated fatty acid or hydroxyl acid having from 8 to 14 carbon atoms linked to the α-amino acid group via an amidic bond;
R₂: is a straight or branched alkyl chain from 1 to 18 carbon atoms or an aromatic group;
R₃: is
—NH₃ 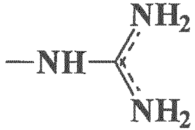
or 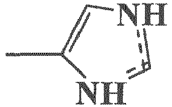
where n is from 0 to 4,
for use in a method of medical treatment for the in-vivo inactivation of endotoxins (lipopolysaccharides, LPS) released from Gram-negative bacteria, exotoxins or aflatoxins.

7. The cationic surfactant for the use according to claim 6 in a method for the treatment of the human or animal body by oral, topical and parenteral administration.

8. The cationic surfactant for the use according to claim 6 in a method for the treatment of the human or animal body by rectal or vaginal administration or by inhalation.

9. The cationic surfactant for the use according to claim 7, wherein the cationic surfactant is administered orally in a dosage of 0.01 to 900 mg/kg bodyweight, preferably of 0.05 to 90 mg/kg bodyweight and more preferably of 0.1 to 9 mg/kg bodyweight.

10. The cationic surfactant for use according to claim 7, wherein the oral administration can consist of a suspension, of an emulsion, of a liquid form such as elixirs and syrups or of a solid form such as capsules, tablets, sachets, lozenges, powder or troches.

11. The cationic surfactant for use according to claim 7, wherein the cationic surfactant is administered parenterally in a dosage of 0.01 to 900 mg/kg bodyweight, preferably of 0.05 to 90 mg/kg bodyweight and more preferably of 0.1 to 9 mg/kg bodyweight.

12. The cationic surfactant for use according to claim 11, wherein the parenteral administration can consist of a sterile liquid form.

13. The cationic surfactant for the use according to claim 6, wherein the cationic surfactant is administered by dermal application in a dosage of 0.01 to 2000 mg/kg bodyweight, preferably of 0.05 to 500 mg/kg bodyweight and the more preferably of 0.1 to 50 mg/kg bodyweight.

14. The cationic surfactant for the use according to claim 13, wherein the dermal administration can consist of a patch mechanism or ointment.

15. The cationic surfactant for the use according to any of claims 6 to 14, wherein the compound is the ethyl ester of the lauramide of arginine hydrochloride (LAE).

## Patentansprüche

1. Verwendung eines kationischen Tensids, welches aus der Kondensation von Fettsäuren und veresterten dibasischen Aminosäuren stammt, gemäß der folgenden Formel (1): 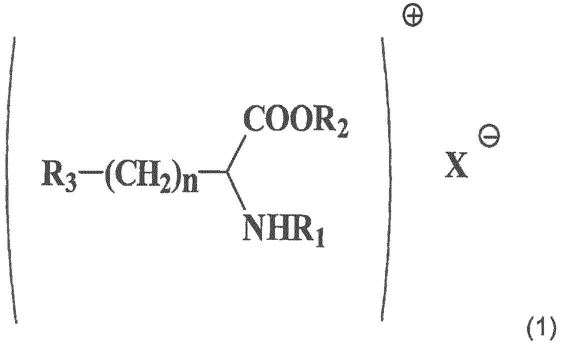 wobei
X⁻ ein Gegenion ist, welches von einer organischen oder anorganischen Säure, vorzugsweise Br⁻, Cl⁻ oder HSO₄⁻, stammt, oder ein Anion auf der Basis einer phenolischen Verbindung;
R₁: eine lineare Alkylkette einer gesättigten Fettsäure oder Hydroxylsäure mit 8 bis 14 Kohlenstoffatomen ist, die an die α-Aminosäuregruppe mittels einer Amidbindung gebunden ist;
R₂: eine lineare oder verzweigte Alkylkette mit 1 bis 18 Kohlenstoffatomen oder eine aromatische Gruppe ist;
R₃:
-NH₃ 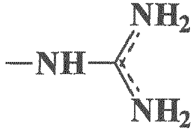
oder 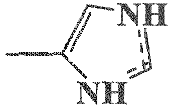
ist, wobei n von 0 bis 4 ist,
zur Inaktivierung von Endotoxinen (Lipopolysacchariden, LPS), die von gramnegativen Bakterien freigesetzt werden, Exotoxinen oder Aflatoxinen in Lebensmittelprodukten und Getränken.

2. Die Verwendung nach Anspruch 1, wobei das kationische Tensid zu einem Lebensmittelprodukt in einer Konzentration von 0,1 bis 100.000 mg/kg des Lebensmittelprodukts, bevorzugt von 1 bis 10.000 mg/kg des Lebensmittelprodukts und am bevorzugtesten von 5 bis 1.000 mg/kg des Lebensmittelprodukts hinzugefügt wird.

3. Verwendung eines kationischen Tensids, welches aus der Kondensation von Fettsäuren und veresterten dibasischen Aminosäuren stammt, gemäß der folgenden Formel (1): 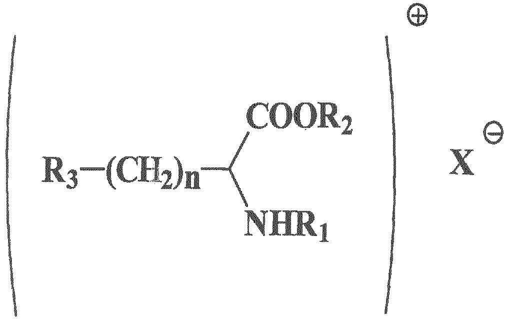 wobei
X⁻ ein Gegenion ist, welches von einer organischen oder anorganischen Säure, vorzugsweise Br⁻, Cl⁻ oder HSO₄⁻, stammt oder ein Anion auf der Basis einer phenolischen Verbindung;
R₁: eine lineare Alkylkette einer gesättigten Fettsäure oder Hydroxylsäure mit 8 bis 14 Kohlenstoffatomen ist, die an die α-Aminosäuregruppe mittels einer Amidbindung gebunden ist;
R₂: eine lineare oder verzweigte Alkylkette mit 1 bis 18 Kohlenstoffatomen oder eine aromatische Gruppe ist;
R₃:
—NH₃ 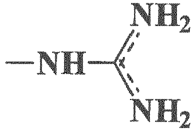
oder 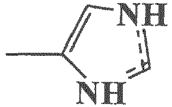
ist, wobei n von 0 bis 4 ist,
zur Inaktivierung von Endotoxinen (Lipopolysacchariden, LPS), die von gramnegativen Bakterien freigesetzt werden, Exotoxinen oder Aflatoxinen, auf Oberflächen von medizinischer Ausrüstung.

4. Die Verwendung nach Anspruch 3 in einer Methode zur Behandlung von medizinischen Geräten durch deren Beschichtung mit kationischen Tensiden, die an deren Oberflächen haften, mit einer Dosierung zwischen 0,2% und 1%.

5. Die Verwendung nach einem der Ansprüche 1 bis 4, wobei die Verbindung der Ethylester des Lauramids von Argininhydrochlorid (LAE) ist.

6. Ein kationisches Tensid, welches aus der Kondensation von Fettsäuren und veresterten dibasischen Aminosäuren stammt, gemäß der folgenden Formel (1): 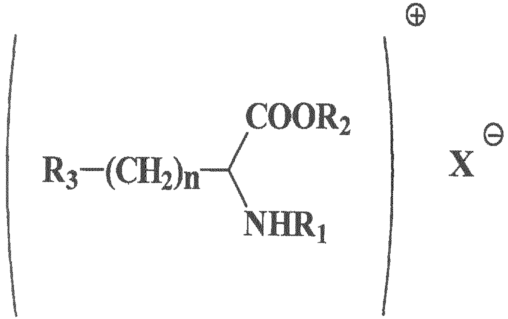 wobei
X⁻ ein Gegenion ist, welches von einer organischen oder anorganischen Säure, vorzugsweise Br⁻, Cl⁻ oder HSO₄⁻, stammt oder ein Anion auf der Basis einer phenolischen Verbindung;
R₁: eine lineare Alkylkette einer gesättigten Fettsäure oder Hydroxylsäure mit 8 bis 14 Kohlenstoffatomen ist, die an die α-Aminosäuregruppe mittels einer Amidbindung gebunden ist;
R₂: eine lineare oder verzweigte Alkylkette mit 1 bis 18 Kohlenstoffatomen oder eine aromatische Gruppe ist;
R₃:
-NH₃ 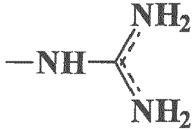
oder 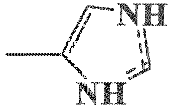
ist, wobei n von 0 bis 4 ist,
zur Verwendung in einer Methode zur medizinischen Behandlung für die in-vivo Inaktivierung von Endotoxinen (Lipopolysacchariden, LPS), die von gramnegativen Bakterien freigesetzt werden, Exotoxinen oder Aflatoxinen.

7. Das kationische Tensid für die Verwendung nach Anspruch 6 in einer Methode zur Behandlung des menschlichen oder tierischen Körpers durch orale, topische und parenterale Verabreichung.

8. Das kationische Tensid für die Verwendung nach Anspruch 6 in einer Methode für die Behandlung des menschlichen oder tierischen Körpers durch rektale oder vaginale Verabreichung oder durch Inhalation.

9. Das kationische Tensid für die Verwendung nach Anspruch 7, wobei das kationische Tensid oral in einer Dosierung von 0,01 bis 900 mg/kg Körpergewicht, bevorzugt von 0,05 bis 90 mg/kg Körpergewicht und am bevorzugten von 0,1 bis 9 mg/kg Körpergewicht verabreicht wird.

10. Das kationische Tensid für die Verwendung nach Anspruch 7, wobei die orale Verabreichung aus einer Suspension, aus einer Emulsion, aus einer flüssigen Form wie Elixiere und Sirupe oder aus einer festen Form wie Kapseln, Tabletten, Sachets, Lutschtabletten, Pulver oder Pastillen bestehen kann.

11. Das kationische Tensid für die Verwendung nach Anspruch 7, wobei das kationische Tensid in einer Dosierung von 0,01 bis 900 mg/kg Körpergewicht, bevorzugt von 0,05 bis 90 mg/kg Körpergewicht und am bevorzugtesten von 0,1 bis 9 mg/kg Körpergewicht parenteral verabreicht wird.

12. Das kationische Tensid für die Verwendung nach Anspruch 11, wobei die parenterale Verabreichung aus einer sterilen flüssigen Form bestehen kann.

13. Das kationische Tensid für die Verwendung nach Anspruch 6, wobei das kationische Tensid durch dermale Anwendung in einer Dosierung von 0,01 bis 2.000 mg/kg Körpergewicht, bevorzugt von 0,05 bis 500 mg/kg Körpergewicht und am bevorzugtesten von 0,1 bis 50 mg/kg Körpergewicht verabreicht wird.

14. Das kationische Tensid für die Verwendung nach Anspruch 13, wobei die dermale Verabreichung aus einem Pflastermechanismus oder einer Salbe bestehen kann.

15. Das kationische Tensid für die Verwendung nach einem der Ansprüche 6 bis 14, wobei der Stoff der Ethylester des Lauramids von Argininhydrochlorid (LAE) ist.

## Revendications

1. Utilisation d'un tensioactif cationique dérivé de la condensation d'acides gras et d'acides aminés dibasiques estérifiés, selon la formule (1) suivante: 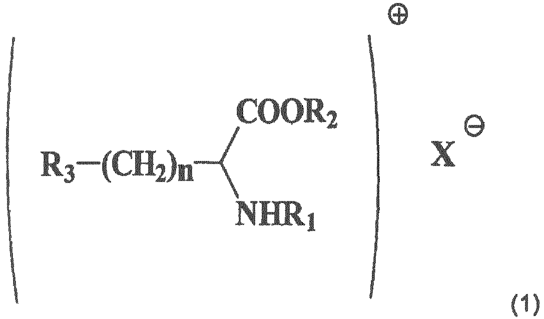 dans laquelle
X⁻ est un contre-ion dérivé d'un acide organique ou inorganique, de préférence Br⁻, Cl⁻ ou HSO₄⁻, ou un anion sur la base d'un composé phénolique ;
R₁ : est une chaîne alkyle droite provenant d'un acide hydroxylé ou d'un acide gras saturé ayant 8 à 14 atomes de carbone liés au groupe acide α-aminé par l'intermédiaire d'une liaison amidique ;
R₂ : est une chaîne alkyle droite ou ramifiée de 1 à 18 atome(s) de carbone ou un groupe aromatique ;
R₃ : est
—NH₃ 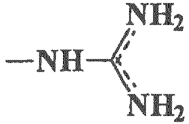
ou 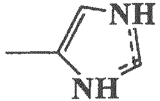
dans laquelle n est compris entre 0 et 4,
pour l'inactivation d'endotoxines (lipopolysaccharides, LPS) libérées par des bactéries Gram-négatives, d'exotoxines ou d'aflatoxines dans les produits alimentaires et les boissons.

2. Utilisation selon la revendication 1, dans laquelle le tensioactif cationique est ajouté à un produit alimentaire à une concentration allant de 0,1 à 100000 mg/kg du produit alimentaire, de préférence allant de 1 à 10000 mg/kg de produit alimentaire et idéalement allant de 5 à 1000 mg/kg du produit alimentaire.

3. Utilisation d'un tensioactif cationique dérivé de la condensation d'acides gras et d'acides aminés dibasiques estérifiés, selon la formule (1) suivante : 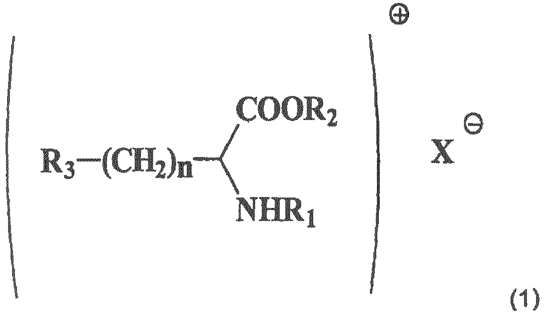 dans laquelle
X- est un contre-ion dérivé d'un acide organique ou inorganique, de préférence Br⁻, Cl⁻ ou HSO₄⁻, ou un anion sur la base d'un composé phénolique ;
R₁ : est une chaîne alkyle droite provenant d'un acide hydroxylé ou d'un acide gras saturé ayant 8 à 14 atomes de carbone liés au groupe acide α-aminé par l'intermédiaire d'une liaison amidique ;
R₂ : est une chaîne alkyle droite ou ramifiée de 1 à 18 atome(s) de carbone ou un groupe aromatique ;
R₃ : est
—NH₃ 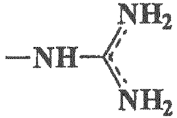
ou 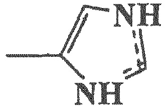
dans laquelle n est compris entre 0 et 4,
pour l'inactivation d'endotoxines (lipopolysaccharides, LPS) libérées par des bactéries Gram-négatives, d'exotoxines ou d'aflatoxines sur des surfaces d'équipement médical.

4. Utilisation selon la revendication 3 dans un procédé de traitement de dispositifs médicaux en les revêtant de tensioactifs cationiques fixés à leurs surfaces à des doses comprises entre 0,2% et 1%.

5. Utilisation selon l'une des revendications 1 à 4, dans laquelle le composé est l'ester éthylique du lauramide de chlorhydrate d'arginine (LAE).

6. Tensioactif cationique dérivé de la condensation d'acides gras et d'acides aminés dibasiques estérifiés, selon la formule (1) suivante : 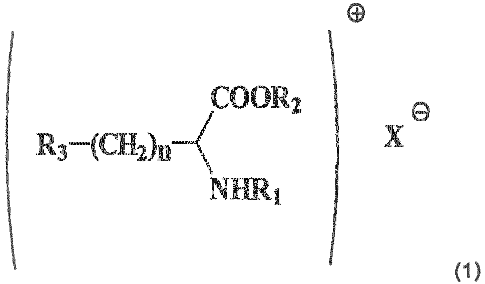 dans laquelle
X⁻ est un contre-ion dérivé d'un acide organique ou inorganique, de préférence Br⁻, Cl⁻ ou HSO₄⁻, ou un anion sur la base d'un composé phénolique ;
R₁ : est une chaîne alkyle droite provenant d'un acide hydroxylé ou d'un acide gras saturé ayant 8 à 14 atomes de carbone liés au groupe acide α-aminé par une liaison amidique ;
R₂ : est une chaîne alkyle droite ou ramifiée de 1 à 18 atome(s) de carbone ou un groupe aromatique ;
R₃ : est
—NH₃ 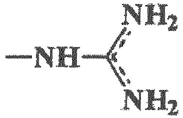
ou 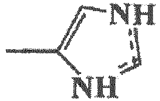
dans laquelle n est compris entre 0 et 4,
pour une utilisation dans un procédé de traitement médical pour l'inactivation in vivo d'endotoxines (lipopolysaccharides, LPS) libérées par des bactéries Gram-négatives, d'exotoxines ou d'aflatoxines.

7. Tensioactif cationique pour une utilisation selon la revendication 6 dans un procédé de traitement du corps humain ou animal par administration orale, topique et parentérale.

8. Tensioactif cationique pour une utilisation selon la revendication 6 dans un procédé de traitement du corps humain ou animal par administration rectale ou vaginale ou par inhalation.

9. Tensioactif cationique pour une utilisation selon la revendication 7, dans lequel le tensioactif cationique est administré par voie orale selon une dose comprise entre 0,01 et 900 mg/kg de poids corporel, de préférence entre 0,05 et 90 mg/kg de poids corporel et plus préférablement entre 0,1 et 9 mg/kg de poids corporel.

10. Tensioactif cationique pour une utilisation selon la revendication 7, dans lequel l'administration orale peut consister en une suspension, en une émulsion, en une forme liquide telle que des élixirs et des sirops, ou en une forme solide telle que des capsules, des comprimés, des sachets, des pastilles, une poudre ou des tablettes.

11. Tensioactif cationique pour une utilisation selon la revendication 7, dans lequel le tensioactif cationique est administré par voie parentérale selon une dose comprise entre 0,01 et 900 mg/kg de poids corporel, de préférence entre 0,05 et 90 mg/kg de poids corporel et plus préférablement entre 0,1 et 9 mg/kg de poids corporel.

12. Tensioactif cationique pour une utilisation selon la revendication 11, dans lequel l'administration parentérale peut consister en une forme liquide stérile.

13. Tensioactif cationique pour une utilisation selon la revendication 6, dans lequel le tensioactif cationique est administré par application dermique selon une dose comprise entre 0,01 et 2000 mg/kg de poids corporel, de préférence entre 0,05 et 500 mg/kg de poids corporel et idéalement entre 0,1 et 50 mg/kg de poids corporel.

14. Tensioactif cationique pour une utilisation selon la revendication 13, dans lequel l'administration dermique peut consister en un système de timbre transdermique ou en une pommade.

15. Tensioactif cationique pour une utilisation selon l'une des revendications 6 à 14, dans lequel le composé est l'ester éthylique du lauramide de chlorhydrate d'arginine (LAE).
